# EUROPEAN PATENT SPECIFICATION

(11) **EP 1 581 229 B1**
(45) Date of publication and mention of the grant of the patent: **12.03.2008**
(21) Application number: 03795950.9
(22) Date of filing: 22.12.2003
(51) Int. Cl.: A61K 31/502, C07D 401/06, C07D 413/14, C07D 409/14, A61P 29/00

(54) **PHTHALAZINE DERIVATIVES AS PHOSPHODIESTERASE 4 INHIBITORS**
PHTHALAZINDERIVATE ALS PHOSPHODIESTERASE-4-HEMMER
DERIVES DE PHTHALAZINE UTILISES COMME INHIBITEURS DE LA PHOSPHODIESTERASE 4

(30) Priority: 23.12.2002 IT MI20022739
(43) Date of publication of application: 05.10.2005
(73) Proprietor: ZAMBON S.p.A., 20091 Bresso MI (IT)
(72) Inventor: NAPOLETANO, Mauro, I-20127 MILANO (IT); PELLACINI, Franco, I-20151 MILANO (IT); MORAZZONI, Gabriele, I-20020 LAINATE (IT); MORIGGI, Jean-Dominique, I-22020 BLEVIO (IT); RIVA, Carlo, I-22044 INVERIGO (IT); HAACK, Thomas, I-22100 COMO (IT); ALLIEVI, Leopoldo, I-22012 CERNOBBIO (IT); PAUSELLI, Marco, I-22078 TURATE (IT)
(86) International application number: PCT/EP2003/014733
(87) International publication number: WO 2004/056366

(56) References cited:
- WO-A-00/05218
- WO-A-99/32456

## Description

The present invention relates to phthalazine derivatives, pharmaceutical compositions containing them and their use as phosphodiesterase 4 inhibitors.

Phosphodiesterases are a family of isoenzymes which constitute the basis of the main mechanism of cAMP (cyclic adenosine-3',5'-monophosphate) hydrolytic inactivation. cAMP has been shown to be the second messenger mediating the biologic response to many hormones, neurotransmitters and drugs [Krebs Endocrinology Proceedings of the 4th International Congress Excerpta Medica, 17-29, 1973]. When the suitable agonist binds to the cell surface, the adenylate cyclase activates and turns Mg²⁺-ATP into cAMP. cAMP modulates the activity of the majority, if not of all the cells contributing to the physiopathology of various respiratory diseases, both of allergic origin and not. It follows that an increase of the cAMP concentration yields beneficial effects such as airway smooth muscle relaxation, inhibition of the mast cell mediator release (basophil granulose cells), suppression of the neutrophil and basophil degranulation, inhibition of the monocyte and macrophage activation. Thus, compounds able of activating adenylate cyclase or of inhibiting phosphodiesterases could suppress the undesired activation of the airway smooth muscle and of a great number of inflammatory cells.

In the phosphodiesterase family there is a distinct group of isoenzymes, phosphodiesterases 4 (hereinafter PDE 4), specific for the hydrolysis of cAMP in the airway smooth muscle and inflammatory cells (Torphy, "Phosphodiesterase Isoenzymes: Potential Targets for Novel Anti-asthmatic Agents" in New Drugs for Asthma, Barnes, ed. IBC Technical Services Ltd, 1989). Studies carried out on this enzyme show that its inhibition yields not only the airway smooth muscle relaxation, but also the suppression of mastocyte, basophil and neutrophil degranulation, so as the inhibition of the monocyte and neutrophil activation. Thus PDE 4 inhibitors are effective in the therapy of asthma.

Selective inhibition of PDE 4 attenuates the functionality of inflammatory cells such as, for example, neutrophils, alveolar macrophages and T cells which possess, as it is known, a key role in COPD (chronic obstructive pulmonary disease) and such activity suggests how this class of compounds could provide an effective therapy in this kind of pathology (Duglas WP Hay, Curr. Opin. Chem. Biol., 2000, vol. 4, pages 412-419).

Such compounds offer a unique approach to the therapy of various respiratory diseases both of allergic origin and not, and possess significant therapeutic advantages over the current therapy.

The excessive or irregular production of tumour necrosis factor (hereinafter TNF_{α}), a cytokine with pro-inflammatory activity produced by various types of cells, affects the mediation or the exacerbation of many pathologies such as, for example, the adult respiratory distress syndrome (ARDS) and the chronic pulmonary inflammatory disease.

Therefore, compounds able to control the negative effects of TNF_{α} i.e. the inhibitors of this cytokine, are to be considered as useful against many pathologies.

The patent application EP 0722936 (in the name of Eisai) claims, *inter alia*, compounds of formula 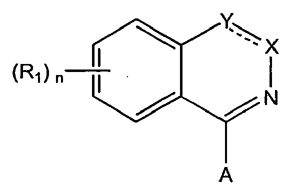 wherein
n = 0-4; R₁ is optionally substituted lower alkoxy, optionally substituted cycloalkyl, or a - OR₉ group wherein R₉ represents an optionally substituted arylalkyl group; X is -N= or - NR₆- wherein R₆ is hydrogen, a lower alkyl group, or optionally substituted arylalkyl or heteroarylalkyl groups; Y is -CO or -CB= wherein B is -NR₇R₈ wherein R₇ and R₈ represent each independently a hydrogen atom, an optionally substituted lower alkyl group, an optionally substituted heteroarylalkyl group or R₇ and R₈ together with the nitrogen atom to which they are bonded may form a ring which may be substituted; or B is a hydrogen atom or an optionally substituted aryl, heteroaryl, arylalkyl or heteroarylalkyl group; A is a hydrogen or halogen atom, or an optionally mono- or disubstituted amino group, an optionally substituted aryl, heteroaryl, arylalkyl or heteroarylalkyl group.

Among the groups that optionally substitute the above mentioned residues, halogen atoms and the optionally protected carboxy group are cited.

The exemplified compounds, included in a very wide general formula, show a predominant interest in the phthalazine nucleus substituted with (3-chloro-4-methoxy)-benzylamino or 3,4-methylenedioxy-benzylamino groups as meanings of A; halogen, nitro groups or cyano with n = 1 as meanings of R₁; a -NR₇R₈ group wherein R₇ and R₈ represent each independently a hydrogen atom, an optionally substituted lower alkyl group, an optionally substituted heteroarylalkyl group or R₇ and R₈ together with the nitrogen atom to which they are bonded may form a ring which may be substituted, as meanings of B.

Therefore, no aspect of the description and no example lead to specific compounds wherein R₁ is methoxy or difluoromethoxy with n = 1, A is phenyl or heterocycle substituted with a carboxy group and optionally with another functional group and B represents a (3,5-dichloro)-pyridin-4-yl-methyl group.

Moreover, these compounds are said to be active as inhibitors of cGMP-PDE, i.e. PDE 5, a phosphodiesterase just acting through a cGMP-dependent mechanism and whose field of application is markedly cardiovascular (Schudt C. et al., Phosphodiesterase Inhibitors, Academic Press).

The international patent application WO 00/05218 (in the name of Zambon Group S.p.A.) claims, *inter alia*, the compounds of formula 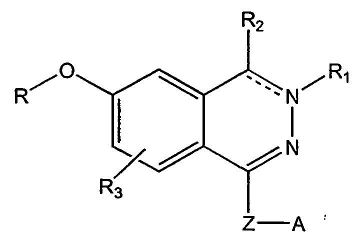 wherein
the bond between the carbon atom to which the substituent R₂ is bonded and the adjacent nitrogen atom is single or double; R is a (C₁-C₆)-alkyl or polyfluoro-(C₁-C₆)-alkyl group; R₁ is absent when the bond between the carbon atom to which the substituent R₂ is bonded and the adjacent nitrogen atom is double or, when the same bond is single, R₁ is a hydrogen atom, an optionally substituted (C₁-C₆)-alkyl group or a (C₁-C₄)-alkylsulfonyl group; R₂ when the bond between the carbon atom to which the substituent R₂ is bonded and the adjacent nitrogen atom is double is a hydrogen atom, a cyano group, amido, (C₁-C₈)-alkyl, (C₂-C₈)-alkenyl or (C₂-C₈)-alkynyl, alkoxy or optionally substituted aryl or heterocycle; R₃ is hydrogen or a (C₁-C₈)-alkyl, (C₂-C₈)-alkenyl or (C₂-C₈)-alkynyl group optionally substituted; Z is NH, methylene or a (C₂-C₆)-alkylene chain optionally branched and/or unsaturated and/or interrupted by a (C₅-C₇)-cycloalkyl residue; A is phenyl or heterocycle optionally substituted by one or more substituent(s).

These compounds are very active as PDE 4 inhibitors and TNF_{α} release inhibitors and, moreover, they do not show activity on PDE 3 and 5 enzymes.

It is evident how this specificity and selectivity of action make these compounds suitable therapeutic agents for the treatment of pathologies involving PDE 4 and TNF_{α}.

Nevertheless, the exemplified compounds of the above patent application, show some physico-chemical characteristics such as, for example, the water-solubility which are suitable for the preparation of a reduced number of compositions.

In fact, the limited solubility of the active principle is the main cause of the difficulties that appear in the preparation of compositions which are able to warrant a sufficiently high bioavailability.

Moreover, in order to obviate the poor solubility in the in vitro and in vivo preclinical models, special compositions that make use of non physiological vehicles and, consequently, that make difficult the evaluation of both the compounds activity and the bioavailability, are commonly used.

It would be desirable, for the use these compounds are intended for, to have a wide range of formulative opportunities for which, in the most classic uses, it is necessary that the active ingredients are sufficiently water-soluble.

The oral route, for example, is the most convenient and widely used method for the administration of drugs and, thus, it results of relevant importance to be able to improve the solubility of potential active ingredients and to permit the development of an oral composition without resorting to special formulative skillness.

We have now surprisingly found that by inserting a carboxy group on the phenyl or heterocycle substituents encompassed in the meanings of R₂ (see WO 00/05218), compounds endowed of a potent inhibitory activity on the PDE 4 and on the TNF_{α} release, inactive on PDE 1, 2, 3 and 5 and, therefore, selective and endowed with optimum water-solubility properties are obtained.

Such compounds constitute a not exemplified sub-class in the ambit of the general formula of the above patent application in the name of Zambon Group and, therefore, they are per se new.

The additional polarity inserted in the molecules by the carboxy group allowed to obtain compounds both endowed with physico-chemical properties more easily manageable from the formulation point of view and to evaluate the bioavailability of the compounds of formula I in vehicles suitable for pharmaceutical compositions; bioavailability that was proved, in an unexpected manner, comparable or in many higher than what found for the compounds of the above cited patent application, which was carried out by dissolving the same compounds in non physiological vehicles.

The insertion of the carboxy group, endowed with a specific polar characteristic, on the phenyl or heterocycle in position 1, allowed the compounds of formula I to be endowed with the correct hydro-lipophilic molecular balance that is necessary to dissolve in fluids and to permeate biological membranes during the absorbption and distribution processes keeping a high inhibitory activity on the PDE 4 enzyme.

Therefore, by the introduction of the carboxyl substituent, compounds endowed with optimum solubility properties, easy to formulate and endowed with inhibitory activity, selectivity and bioavailability which are comparable to or superior to the compounds of which they constitute a selection, were obtained.

Therefore, object of the present invention are compounds of formula 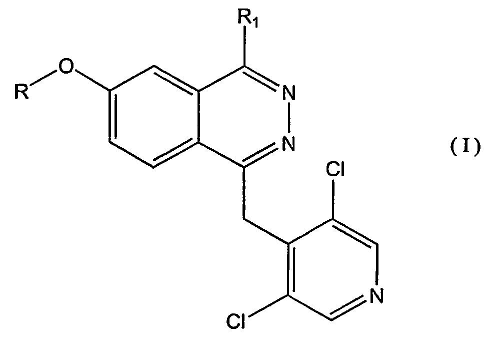 wherein
R is methyl or a difluoromethyl group;
R₁ is phenyl or a 5 or 6 membered aromatic heterocycle containing from 1 to 3 heteroatoms selected among nitrogen, oxygen and sulphur and bonded to the phthalazine nucleus by a carbon-carbon bond, both the phenyl and the heterocycle being substituted with a carboxy group and optionally with a second functional group selected among methoxy, nitro, N-acetylamino, N-methanesulfonyl-amino;
the N-oxidised derivatives of the compounds of formula I and the pharmaceutically acceptable salts thereof.

The compounds of formula I are active as PDE 4 and TNF_{α} inhibitors and thus they find a used as therapeutic agents in allergic and inflammatory pathologies such as, for example, ARDS, COPD, asthma and allergic rhinitis.

With the term 5 or 6 membered aromatic heterocycle containing from 1 to 3 heteroatoms selected among nitrogen, oxygen and sulphur, aromatic heterocycle such as pyrrole, thiophene, furan, imidazole, pyrazole, thiazole, isothiazole isoxazole, oxazole, pyridine, pyrazine, pyrimidine, pyridazine, triazole and thiadiazole are meant.

The N-oxidised form, if it is present, may involve both the nitrogen atoms present on the phthalazine ring and that present on the pyridyl ring.

Pharmaceutically acceptable salts of the compounds of formula I are salts with alkaline or alkaline-earth metals, zinc salts and salts with pharmaceutically acceptable organic bases such as, for example, trometamol (2-amino-2-hydroxymethyl-propane-1,3-diol), N-methyl-glucamine.

Preferred compounds of formula I are those compounds wherein R₁ is phenyl substituted with a carboxyl group and particularly, those compounds wherein the carboxyl group is in meta position with regard to the phthalazine nucleus.

Specific examples of compounds object of the invention are:
3-[4-(3,5-Dichloro-pyridin-4-ylmethyl)-7-methoxy-phthalazin-1-yl]-benzoic acid;
4-[4-(3,5-Dichloro-pyridin-4-ylmethyl)-7-methoxy-phthalazin-1-yl]-benzoic acid;
2-[4-(3,5-Dichloro-pyridin-4-ylmethyl)-7-methoxy-phthalazin-1-yl]-benzoic acid;
3-[4-(3,5-Dichloro-pyridin-4-ylmethyl)-7-methoxy-phthalazin-1-yl]-5-nitro-benzoic acid;
5-[4-(3,5-Dichloro-pyridin-4-ylmethyl)-7-methoxy-phthalazin-1-yl]-2-methoxy-benzoic acid;
3-[4-(3,5-Dichloro-pyridin-4-ylmethyl)-7-methoxy-phthalazin-1-yl]-4-methoxy-benzoic acid;
3-[4-(3,5-Dichloro-pyridin-4-ylmethyl)-7-methoxy-phthalazin-1-yl]-5-methanesulfonyl-amino-benzoic acid;
3-Acetylamino-5-[4-(3,5-dichloro-pyridin-4-ylmethyl)-7-methoxy-phthalazin-1-yl]-benzoic acid;
3-[4-(3,5-Dichloro-pyridin-4-ylmethyl)-7-methoxy-phthalazin-1-yl]-5-methoxy-benzoic acid;
3-[4-(3,5-Dichloro-pyridin-4-ylmethyl)-7-difluoromethoxy-phthalazin-1-yl]-benzoic acid;
2-[4-(3,5-Dichloro-pyridin-4-ylmethyl)-7-methoxy-phthalazin-1-yl]-oxazole-4-carboxylic acid;
2-[4-(3,5-Dichloro-pyridin-4-ylmethyl)-7-methoxy-phthalazin-1-yl]-thiophene-3-carboxylic acid;
(2-amino-2-hydroxymethyl-propane-1,3-diol) salt of 3-[4-(3,5-Dichloro-pyridin-4-ylmethyl)-7-methoxy-phthalazin-1-yl]-benzoic acid;
N-methyl-glucamine salt of 3-[4-(3,5-Dichloro-pyridin-4-ylmethyl)-7-methoxy-phthalazin-1-yl]-benzoic acid.

The compounds of formula I, object of the present invention, are prepared by an aromatic nucleophilic substitution reaction or a coupling reaction, in presence of a catalyst such as for example palladium, between a compound of formula 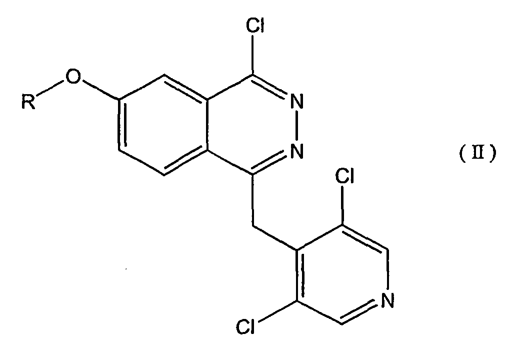 wherein
R has the meanings reported for the compounds of formula I;
and a reactant such as a tin or boronic acid derivative, suitable for the substitution of the halogen atom directly bonded to the phthalazine nucleus with a phenyl or a heterocycle substituted with a carboxy group and optionally substituted with a second functional group defined in the meanings of R₁ in formula I.

The phthalazine derivatives of formula II are prepared according to the synthetic scheme described in the international patent application WO 00/05218 (in the name of Zambon Group S.p.A.) example 45, page 35, and example 99, page 57.

In case tin derivatives in the coupling reaction are used, it would be better that they contain precursors of the free carboxy group such as, for example, a cyano group or an ester group.

Preferably, a Suzuki coupling reaction between the compounds of formula II and the proper boronic acid is carried out in presence of palladium, triphenylphosphine and an aqueous solution of potassium carbonate.

The used boronic acids are, for example, optionally substituted carboxyl-(phenyl or heterocycle)-boronic acids or optionally substituted formyl-(phenyl or heterocycle)-boronic acids which are then oxidised to give the corresponding compounds of formula I.

As an alternative, for the derivatives wherein R₁ is a substituted heterocycle, the heteroaromatic ring is built up by a multistep reaction, according to common synthetic techniques, using as substrate the suitably functionalised phthalazine nucleus.

The preparation of the N-oxydised compounds of formula I is carried out by treatment with peracids such as, for example, m-chloroperbenzoic acid.

In case the oxidation reaction is directed to the nitrogen atom present on the pyridine ring, in order to warrant selectivity to the process, this is carried out on the isobenzofuranol nucleus, precursor of the phthalazine nucleus, according to what described in the international patent application WO 00/05218.

The preparation of the salts of the compounds of formula I is carried out according to known methods.

The compounds of formula I are PDE 4 inhibitors as shown by the enzymatic inhibitory tests (Example 18), and are also able to inhibit the TNF_{α} release (Example 19).

Furthermore, the compounds of the present invention do not show any inhibitory activity on PDE 1, 2, 3 and 5 enzymes as shown by the enzymatic inhibition tests carried out.

It is evident how these enzimatic selectivity and specificity characteristics combined with the lack of activity on the cardiovascular system make the compounds of formula I specifically suitable for treating pathologies involving PDE 4 and TNF_{α} such as asthma, COPD, ARDS, allergic rhinoconjunctivitis, psoriasis, atopic dermatitis, rheumatoid arthritis, septic shock, ulcerative cholitis, even if in the present contest the interest is particularly focused on the respiratory pathologies. In particular, the compounds of the invention are useful in the treatment of allergic and inflammatory diseases and particularly in the therapy of ARDS, COPD, asthma and allergic rhinitis.

The therapeutic doses are generally comprised from 0.1 to 1.000 mg/day and from 1 to 200 mg by oral route for single administration.

The therapeutically effective amounts will depend on the age and on the general physiological conditions of patient, on the administration route and on the used pharmaceutical composition.

The compounds of the present invention for their therapeutic or preventive use in the above mentioned pathologies will be preferably used in a pharmaceutical composition suitable for the oral, rectal, sublingual, parenteral, topical, transdermal and inhalatory administration Therefore, a further object of the present invention are pharmaceutical compositions containing a therapeutically effective amount of a compound of formula I or a salt thereof in admixture with a pharmaceutically acceptable carrier.

The pharmaceutical compositions object of the present invention may be liquid, suitable for the oral and/or parenteral administration such as, for example, drops, sirups, solutions, injectable solutions ready to use or prepared by the dilution of a lyophilized preparation, and solid or semisolid such as tablets, capsules, granulates, powders, pellets, vaginal suppositories, suppositories, creams, ointments, gels, unguents; or still solutions, suspensions, emulsions, and other forms suitable for the inhalatory or transdermal administrations.

Depending on the type of composition, besides a therapeutically effective amount of one (or more) compounds of formula I, they will contain some solid or liquid excipients or diluents for pharmaceutical use and optionally further additives, commonly used in the preparation of pharmaceutical compositions, such as thickeners, binders, lubricants, disintegrators, flavouring and coloring agents.

The preparation of the pharmaceutical compositions object of the invention can be carried out according to common techniques.

For better illustrating the invention the following examples are now given.

### Example 1

### Synthesis of 4-[4-(3,5-dichloro-pyridin-4-ylmethyl)-7-methoxy-phthalazin-1-yl]-benzoic acid (compound 1)

To a mixture of 4-chloro-1-(3,5-dichloro-pyridin-4-ylmethyl)-6-methoxy-phthalazine (intermediate 1) (300 mg, 0.85 mmol, 1 eq.), prepared according to what described in the international patent application WO 00/05218 example 45 page 35, 4-carboxyphenylboronic acid (155 mg, 0.93 mmol, 1.1 eq.) and palladium tetrakis(triphenylphosphine) (50 mg, 5 mol %), a 2 N aqueous solution of potassium carbonate (1.26 ml), DME (10 ml) and ethanol (0.5 ml) previously flushed with nitrogen were added at room temperature under an inert atmosphere of argon. The reaction mixture was stirred at 90°C for from 4 to 16 hours. The reaction was quenched with a 5% aqueous solution of citric acid until pH=6. The coupling reaction product was extracted with ethyl acetate (2 x 15 ml), dried (Na₂SO₄) and concentrated under reduced pressure to give compound 1 as a pale yellow solid (216 mg, 0.49 mmol, 58% yield).
MS: 440 [M+H]⁺
NMR DMSO_d6: 8.70 (s, 2H, Py); 8.58 (d, 1H, JHH=9.1 Hz, *CH=CH-C-OMe); 8.14 (d-broad, 2H, JHH=8.3 Hz, C-(*CH=CH)2-C), 7.87 (d-broad, 2H, JHH=8.3 Hz, C-(CH=*CH)2-C); 7.79 (dd, 1H, CH=*CH-C-OMe); 7.29 (d, 1H, JHH=2.5 Hz, C=*CH-C); 5.04 (s, 2H, *CH2-Py); 3.89 (s, 3H, OMe).

### Example 2

### Synthesis of 3-[4-(3,5-dichloro-pyridin-4-ylmethyl)-7-methoxy-phthalazin-1-yl]-benzoic acid (compound 2);

Compound 2 was synthetised, operating as described for compound 1, by using intermediate 1 (5 g, 14 mmol) and 3-carboxyphenylboronic acid (2.5 g, 15 mmol) as substrates. The coupling reaction product was extracted with ethyl acetate (2 x 15 ml), dried (Na₂SO₄) and then filtered over celite, eluting with ethyl acetate (2 x 30 ml). The filtrate was acidified with concentrated hydrochloric acid until pH=4 and stirred for one hour. The precipitate was collected by filtration and dried at 50°C to give compound 2 as a pale yellow solid (3.4 g, 8 mmol, 57% yield).
MS: 440 [M+H]⁺
NMR DMSO_d6: 13.20 (s-broad, 1H, OH); 8.71 (s, 2H, Py); 8.58 (d, 1H, JHH=8.7 Hz, *CH=CH-C-OMe); 8.25 (s-broad, 1H, C=*CH-C-CO2H); 8.14 (d-broad, 1H, JHH=8.1 Hz, *CH), 8.02 (d-broad, 1H, JHH=8.1 Hz, *CH); 7.80 (dd, 1H, CH=*CH-C-OMe); 7.77-7.69 (m, 1H, CH=*CH-CH); 7.29 (d, 1H, JHH=2.3 Hz, C=*CH-C); 5.04 (s, 2H, *CH2-Py); 3.88 (s, 3H, OMe).

### Example 3

### Synthesis of 3-[4-(3,5-dichloro-pyridin-4-ylmethyl-7-methoxy-phthalazin-1-yl]-5-nitrobenzoic acid (compound 3)

Compound 3 was synthetised, operating as described for compound 1, by using intermediate 1 (300 mg, 0.85 mmol) and 3-carboxy-5-nitro-phenylboronic acid (196 g, 0.93 mmol) as substrates. The coupling reaction product was extracted with ethyl acetate (2 x 15 ml), dried (Na₂SO₄), concentrated under reduced pressure and then filtered over celite, eluting with ethyl acetate (10 ml). The filtrate was concentrated under reduced pressure to give compound 3 as a pale yellow solid (235 mg, 0.48 mmol, 57% yield).
MS: 485 [M+H]⁺
NMR DMSO_d6: 8.78-8.73 (m, 2H, *CH,*CH); 8.71 (s, 2H, Py); 8.64-8.60 (m, 2H, *CH=CH-C-OMe, *CH); 8.84 (dd, 1H, JHH=9.2 Hz, 2.5 Hz, CH=*CH-C-OMe); 7.32 (d, 1H, JHH=2.5 Hz, MeO-C=*CH-C); 5.07 (s, 2H, *CH2-Py); 3.89 (s, 3H, OMe).

### Example 4

### Synthesis of 5-[4-(3,5-dichloro-pyridin-4-ylmethyl)-7-methoxy-phthalazin-1-yl]-2-methoxy-benzaldeyde (intermediate 2)

Intermediate 2 was synthetised, operating as described for compound 1, by using intermediate 1 (450 mg, 1.26 mmol) and 2-formyl-4-methoxy-phenylboronic acid (248 g, 1.38 mmol) as substrates. The reaction mixture was poured into a Varian ChemElut CE100S and washed with dichloromethane (3 x 10 ml). The filtrate was concentrated under reduced pressure and the product was purified on a Varian Mega Bond ChemElut (SiO₂) eluting with CH₂Cl₂/methanol (100:0 to 97:3 v/v). Finally the product was recrystallised from methanol to give a pale yellow solid as intermediate 2 (260 mg, 0.6 mmol, 48% yield).
MS: 455 [M+H]⁺
NMR DMSO_d6: 10.40 (s, 1H, CHO); 8.70 (s, 2H, Py); 8.57 (d, 1H, JHH=9.1 Hz, *CH=CH-C-OMe); 8.10 (dd, 1H, JHH=2.3 Hz, 8.5 Hz, C-*CH=CH-C (OMe)-C-CHO); 8.04 (d, 1H, JHH=2.3 Hz, *CH-C-CHO); 7.79 (dd, 1H, JHH=2.5 Hz, 9.1 Hz, CH-*CH=C-OMe); 7.46 (d, 1H, JHH=8.7 Hz, *CH=C(OMe)-C-CHO); 7.32 (d, 1H, JHH=2.6 Hz, C=*CH-C); 5.02 (s, 2H, *CH2-Py); 4.04 (s, 3H, OMe); 3.89 (s, 3H, OMe).

### Example 5

### Synthesis of 5-[4-(3,5-dichloro-pyridin-4-ylmethyl)-7-methoxy-phthalazin-1-yl]-2-methoxybenzoic acid (compound 4)

To a rapidly stirred solution of intermediate 2 (70 mg, 0.15 mmol, 1 eq.) in tert-butyl alcohol (3 ml) and a 2 N solution of 2-methyl-2-butene in THF (0.77 ml, 1.5 mmol, 10 eq.), a solution of sodium chloride (16 mg, 0.18 mmol, 1.2 eq.) in an 1 N aqueous potassium dihydrogen phosphate buffer (pH = 3.5, 1 ml, 1.12 mmol, 7.5 eq.) was added dropwise at room temperature under an inert nitrogen atmosphere. The reaction mixture was stirred at room temperature for 5 hours and concentrated under reduced pressure. The resulting material was diluted with water (5 ml). The product was extracted with dichloromethane (2 x 7 ml) and concentrated under reduced pressure. The product was purified on a Varian Mega Bond ChemElut (SiO₂) eluting with CH₂Cl₂/methanol (100:0 to 97:3 v/v). The purification was followed by elution with 100% methanol to give compound 4 as a yellow solid (47 mg, 0.1 mmol, 67% yield).
MS: 471 [M+H]⁺
NMR DMSO_d6: 8.69 (s, 2H, Py); 8.53 (d, 1H, JHH=9.3 Hz, *CH=CH-C-OMe); 7.80-7.70 (m, 3H, C-C-CH=*CH-C, C=*CH-C-COOH, CH=*CH-C-OMe); 7.36 (d, 1H, JHH=2.5 Hz, C=*CH-C); 7.20 (d, 1H, JHH=8.7 Hz, C-C-*CH=CH-C); 5.00 (s, 2H, *CH2-Py); 3.88 (s, 3H, OMe); 3.85 (s, 3H, OMe).

### Example 6

### Synthesis of 3-[4-(3,5-dichloro-pyridin-4-ylmethyl)-7-methoxy-phthalazin-1-yl]-4-methoxy-benzaldeyde (intermediate 3)

Intermediate 3 was synthetised, operating as described for compound 1, by using intermediate 1 (450 mg, 1.26 mmol) and 5-formyl-2-methoxy-phenylboronic acid (248 g, 1.38 mmol) as substrates. The reaction mixture was poured into a Varian ChemElut CE100S and washed with dichloromethane (3 x 10 ml). The filtrate was concentrated under reduced pressure and the product was purified on a Varian Mega Bond ChemElut (SiO₂) eluting with CH₂Cl₂/methanol (100:0 to 97:3 v/v). Finally the product was recrystallised from methanol to give a pale yellow solid as intermediate 3 (214 mg, 0.5 mmol, 40% yield).
MS: 455 [M+H]⁺
NMR DMSO_d6: 9.96 (s, 1H, CHO); 8.71 (s, 2H, Py); 8.54 (d, 1H, JHH=9.2 Hz, C=C-*CH=CH-C-OMe); 8.15 (dd, 1H, JHH=2.3 Hz, 8.5 Hz, CHO-C-*CH=CH); 7.94 (d, 1H, JHH=2.3 Hz, C-*CH=C-CHO); 7.75 (dd, 1H, JHH=2.6 Hz, 9.2 Hz, C=C-CH=*CH-C-OMe); 7.47 (d, 1H, JHH=8.5 Hz, C-*CH=CH-C-CHO); 6.83 (d, 1H, JHH=2.3 Hz, C=*CH-COMe); 5.05 (s, 2H, *CH2-Py); 3.83 (s, 3H, OMe); 3.82 (s, 3H, OMe).

### Example 7

### Synthesis of 3-[4-(3,5-dichloro-pyridin-4-ylmethyl)-7-methoxy-phthalazin-1-yl]-4-methoxybenzoic acid (compound 5)

Compound 5 was synthetised, operating as described for compound 4, by using intermediate 3 (150 mg, 0.33 mmol) as substrate. The product was purified on a Varian Mega Bond ChemElut (SiO₂) eluting with CH₂Cl₂/methanol/acetic acid (99:1:0.05 v/v/v) to give compound 5 as a yellow solid (78 mg, 0.16 mmol, 50% yield).
MS: 470 [M+H]⁺
NMR DMSO_d6: 8.70 (s, 2H, Py); 8.53 (d, 1H, JHH=9.1 Hz, *CH=CH-C-OMe); 8.16 (dd, 1H, JHH=2.2 Hz, 8.7 Hz, CH-*CH=C-COOH); 7.90 (d, 1H, JHH=2.2 Hz, C-*CH-C-CO2H); 7.74 (dd, 1H, JHH=2.5 Hz, 9.1 Hz, CH=*CH-C-OMe); 7.36 (d, 1H, JHH=8.7 Hz, *CH-CH=C-COOH); 6.82 (d, 1H, JHH=2.5 Hz, C=*CH-C-OMe); 5.04 (s, 2H, *CH2-Py); 3.81 (s, 3H, OMe); 3.79 (s, 3H, OMe).

### Example 8

### Synthesis of 3-[4-(3,5-dichloro-pyridin-4-ylmethyl)-7-methoxy-phthalazin-1-yl]-5-methanesulfonylamino-benzoic acid (compound 6)

3-Carboxy-5-nitrophenylboronic acid (250 mg, 1.1 mmol) was added to a suspension of palladium on charcoal (12 mg) in methanol (15 ml) and reduced in an atmosphere of hydrogen (40 psi). After 1.5 hours, the reaction mixture was filtered over celite and the filtrate concentrated under reduced pressure. The crude (200 mg, about 1.1 mmol) was then dissolved in water (2 ml) and dioxane (1 ml). A 10 N aqueous solution of sodium hydroxide (0.33 ml, 3 eq.) was added. To the mixture a solution of methanesulfonyl chloride (0.1 ml, 1.2 mmol, 1.1 eq.) in dichloromethane (1 ml) at 0°C was added dropwise. The reaction mixture was then stirred at room temperature for 2 hours and acidified with a 5% citric acid aqueous solution. The product was extracted with ethyl acetate (2 x 10 ml). The combined organic layers were dried (Na₂SO₄) and concentrated under reduced pressure. The crude (130 mg, 0.5 mmol) was then coupled with intermediate 1 (178 mg, 0.5 mmol) under the Suzuki conditions operating as described for compound 1. The product was extracted with ethyl acetate (2 x 15 ml), dried (Na₂SO₄) and concentrated under reduced pressure. The product was purified on a Varian Mega Bond ChemElut (SiO₂) eluting with CH₂Cl₂/methanol/acetic acid (99:1:0.05 v/v/v) to give compound 6 as a pale yellow solid (13 mg, 0.02 mmol, 5% yield).
MS: 534 [M+H]⁺
HPLC/MS: Gilson instrument equipped with a C18 column Zorbax SBC18 (3.5 µm, 2.1 x 50 mm) coupled with a diode array UV detector (220 nm) and a Finnigan Aqa mass spectrometer (electron spray, positive ionization). The following settings were used: flow rate: 1 mL/min; column temperature: 40°C; gradient elution A/B (eluent A: 0.5% formic acid in water; eluent B: 0.5% formic acid in acetonitrile); t = 0 min., A/B = 95:5, t = 8 min., A/B = 5:95; Rt 4.36 min.

### Example 9

### Synthesis of 3-[4-(3,5-dichloro-pyridin-4-ylmethyl)-7-methoxy-phthalazin-1-yl]-5-methoxybenzoic acid methyl ester (intermediate 4)

To a suspension of 3,5-dinitrobenzoic acid ethyl ester (5 g, 0.02 mol, 1 eq.) in methanol (50 ml) under an inert nitrogen atmosphere methyl lithium (1.66 g, 0.04 mol, 2.1 eq.) was added. The reaction mixture was heated at reflux temperature for 16 hours and poured into an ice-cold aqueous solution of hydrochloric acid (25 ml). The product was extracted with ether (3 x 25 ml), washed with water (10 ml), dried (Na₂SO₄) and concentrated under reduced pressure. The product was purified on a Varian Mega Bond ChemElut (SiO₂) eluting with cyclohexane/ethyl acetate (9:1 v/v).

The resulting 3-methoxy-5-nitro-benzoic acid methyl ester (1 g, 5 mmol) was dissolved in methanol (30 ml) and ethyl acetate (20 ml). A concentrated aqueous solution of hydrochloric acid (0.4 ml) and palladium on charcoal (50 mg) were added. The reaction mixture was kept under a hydrogen atmosphere (40 psi) at room temperature. After 1 hour, the palladium catalyst was collected by filtration over celite and the filtrate concentrated under reduced pressure. The resulting 3-amino-5-methoxy-benzoic acid methyl ester was used without any further purification. To a suspension of crude 3-amino-5-methoxy-benzoic acid methyl ester (960 mg, 4.4 mmol, 1 eq.) in a 1:1 (v/v) mixture of water and concentrated hydrochloric acid (5 ml) at 0°C, a solution of NaNO₂ (365 mg, 5.3 mmol, 1.2 eq.) in water (5 ml) was added dropwise. The mixture was stirred for 10 minutes at 0°C. A solution of potassium iodide (1.47 g, 8.8 mmol, 2 eq.) in water (3 ml) was then slowly added. Simultaneously toluene (8 ml) was poured into the reaction mixture. The ice bath was removed and the reaction mixture stirred at room temperature for 3 hours, then at reflux temperature for 1 hour. Water (10 ml) was added and the product was extracted with ethyl acetate (2 x 10 ml). The combined organic layers were washed with brine (5 ml), dried (Na₂SO₄) and concentrated under reduced pressure. Purification by flash chromatography (SiO₂) eluting with cyclohexane/ethyl acetate 9:1 (v/v) afforded 3-iodo-5-methoxy-benzoic acid methyl ester (730 mg, 2.5 mmol, 50% two steps yield).

3-Iodo-5-methoxy-benzoic acid methyl ester (500 mg, 1.7 mmol, 1 eq.) was added to a solution of tetrakis(triphenylphosphine) palladium (20 mg, 4 mol%) in toluene (25 ml) under an inert argon atmosphere. To the reaction mixture hexamethyldistannate (0.41 ml, 2 mmol, 1.2 eq.) was added and the mixture was heated to reflux temperature. After 3 hours, ethyl acetate (10 ml) was added. The reaction mixture was washed with a pH=7 NaOH/KH₂PO₄ aqueous buffer (15 ml), dried (Na₂SO₄) and concentrated under reduced pressure. The resulting product was diluted with toluene (30 ml) and added to a mixture of intermediate 1 (610 mg, 1.7 mmol, 1 eq.) and palladium tetrakis(triphenylphosphine) (90 mg, 5 mol%) under an inert argon atmosphere. The mixture was heated at 110°C for 18 hours. The product was extracted with ethyl acetate (2 x 15 ml), dried (Na₂SO₄) and concentrated under reduced pressure and was purified by flash chromatography (SiO₂) eluting with cyclohexane/ethyl acetate/methanol 50:50:1 (v/v/v) affording the intermediate 4 as pale yellow solid (483 mg, 1 mmol, 60% yield).
MS: 484 [M+H]⁺
NMR DMSO_d6: 8.71 (s, 2H, Py); 8.59 (d, 1H, JHH=9.2 Hz, *CH=CH-C-OMe); 7.84-7.81 (m, 1H, *CH); 7.78 (d, 1H, JHH=2.6 Hz, *CH); 7.64-7.59 (m, 2H, *CH, *CH); 7.31 (d, 1H, JHH=2.5 Hz, C-C=*CH-C); 5.04 (s, 2H, *CH2-Py); 3.90 (s, 3H, OMe); 3.89 (s, 3H, OMe); 3.88 (s, 3H, OMe).

### Example 10

### Synthesis of 3-[4-(3,5-dichloro-pyridin-4-ylmethyl)-7-methoxy-phthalazin-1-yl]-5-methoxybenzoic acid (compound 7)

To a solution of intermediate 4 (430 mg, 0.9 mmol, 1 eq.) in a 3:1 (v/v) THF/water mixture (28 ml) lithium hydroxide (65 mg, 2.7 mmol, 3 eq.) was added at room temperature. The reaction mixture was stirred for 4 hours and poured into ice-cold water (10 ml). The mixture was acidified with a 5% aqueous solution of citric acid and the product was extracted with ethyl acetate (2 x 10 ml). The combined organic layers were dried (Na₂SO₄) and concentrated under reduced pressure to give the compound 7 as a pale yellow solid without any purification (420 mg, 0.9 mmol, 100% yield).
MS: 471 [M+H]⁺
NMR DMSO_d6: 13.21 (s-broad, 1H, OH); 8.70 (s, 2H, Py); 8.58 (d, 1H, JHH=9.1 Hz, *CH=CH-C-OMe); 7.82-7.80 (m, 1H, *CH); 7.77 (d, 1H, JHH=2.5 Hz, *CH); 7.63-7.61 (m, 1H, *CH); 7.56-7.54 (m, 1H, *CH); 7.31 (d, 1H, JHH=2.3 Hz, C-C=*CH-C); 5.03 (s, 2H, *CH2-Py); 3.88 (s, 6H, OMe, OMe).

### Example 11

### Synthesis of 3-[4-(3,5-dichloro-pyridin-4-ylmethyl)-7-difluoromethoxy-phthalazin-1-yl]-benzoic acid (compounds 8)

To a mixture of the compound 4-chloro-1-(3,5-dichloro-pyridin-4-ylmethyl)-6-difluoromethoxy-phthalazine (5 g, 12.8 mmol, 1eq.), prepared according to what described in the international patent application WO 00/05218 example 99 page 45, 3-carboxyphenylboronic acid (2.34 g, 14 mmol, 1.1 eq.) and palladium tetrakis(triphenylphosphine) (450 mg, 5 mol %) under an inert argon atmosphere a solution of potassium carbonate (5.3 g, 39 mmol, 3 eq.) in water (30 ml), DME (100 ml) and ethanol (10 ml) previously flushed with nitrogen was added at room temperature. The reaction mixture was stirred at 80°C for 16 hours. The reaction mixture was concentrated under reduced pressure and water was added (250 ml). Excess reactant was extracted with ethyl acetate (3 x 50 ml) while the aqueous layer was acidified with concentrated hydrochloric acid to pH=3. The mixture was stirred for 1 hour. The precipitate was collected by filtration and dried at 50°C to give compound 8 as a pale yellow solid. Further recrystallisation from chloroform afforded a purer compound 8 (3.9 g, 8.7 mmol, 68% yield).
MS: 477 [M+H]⁺
NMR DMSO_d6: 8.77 (d, 1H, JHH=9.0 Hz, *CH=CH-C-O-CHF₂); 8.72 (s, 2H, Py); 8.24 (m, 1H, *CH=C-COH); 8.19-7.97 (m, 3H, *CH=CH-CH=C-COOH, CH=*CH-C-COOH, CH=*CH-C-OCHF₂); 7.74 (m, 1H, CH-*CH=CH); 7.61 (d, 1H, JHH=2.4 Hz, CHF2-O-C-*CH=C); 7.51 (t, 1H, JHF=73.4 Hz, *CHF₂); 5.10 (s, 2H, *CH2-Py).

### Example 12

### Synthesis of 1-[4-(3,5-dichloro-pyridin-4-ylmethyl)-7-methoxy- phthalazin-1-yl]-carboxyl acid methyl ester (intermediate 5)

To a suspension of intermediate 1 (9.7 g, 27 mmol) in DMSO (80 ml) and methanol (40 ml), potassium carbonate (7.4 g, 54 mmol), palladium acetate (0.31 g, 1.4 mmol) and 1,3-bisdiphenylphosphine propane (0.75 g, 1.8 mmol) were added. The reaction mixture was kept under a carbon monoxide (8.5 bar) atmosphere and was warmed to 50°C for hours. The reaction was poured into water (1 1). The product was extracted with ethyl acetate (4 x 200 ml). It was washed with a saturated solution of brine (300 ml), dried and concentrated under vacuum. The product was purified by a flash chromathography (SiO₂) eluting with petroleum ether/ethyl acetate (1:1 v/v) to give intermediate 5 as white solid (5.0 g, 13 mmol, 49% yield).
NMR CDCl3: 8.42 e 7.97 (2s, 2H, Py); 7.90 (d, 1H, JHH=8.6 Hz, *CH=CH-C-OMe); 7.31-7.23 (m, 2H, Ar); 7.19 (dd, 1H, CH-*CH=C-OMe); 7.11-7.09 (m, 2H, Ar); 7.02 (d, 1H, JHH=2.3 Hz, C=*CH-C=CH); 5.62 (s, 1H, CH); 5.08 (s, 1H, CH); 3.95 (s, 4H, OCH2CH2O); 3.91 (s, 3H, OCH3); 3.42 (bs, 1H, OH).

### Example 13

### Synthesis of 1-[4-(3,5-dichloro-pyridin-4-ylmethyl)-7-methoxy₋phthalazin-1-yl]-carboxylic acid (intermediate 6)

To a suspension of intermediate 5 (750 mg, 1.98 mmol) in methanol (27 ml) and water (3 ml), 85% potassium hydroxide (210 mg, 3 mol) was added. After 1 hour, the reaction mixture was concentrated and water (10 ml) was added. It was washed with dichloromethane (2 x 5 ml). It was acidified with a concentrated aqueous solution of HCl. The obtained precipitate was filtered and washed with water (2 x 5 ml) to give intermediate 6 as white solid (0.66 g, 1.8 mmol, 92% yield).
NMR DMSO_d6: 10.13 (s, 1H, CHO); 8.70 (s, 2H, Py); 8.57 (d, 1H, JHH=9.0 Hz, *CH=CH-C-OMe); 8.29-7.80 (m, 4H, Ar-CHO); 7.82-7.76 (dd, 1H, CH-*CH=C-Ome); 7.28 (d, 1H, JHH=2.65 Hz, MeO-C-*CH=C); 4.96 (s, 2H, *CH2-Py); 3.88 (s, 3H, OMe).

### Example 14

### Synthesis of 2-[4-(3,5-dichloro-pyridin-4-ylmethyl)-7-methoxy-phthalazin-1-yl]-oxazole-4-carboxylic acid (compounds 9)

To a solution of serine ethyl ester hydrochloride (233 mg, 1.4 mmol, 1eq.) in DMF (3 ml) a solution of intermediate 6 (500 mg, 1.4 mmol, 1 eq.) in dichloromethane (15 ml), triethylamine (0.22 ml, 1.5 mmol, 1.1 eq.) and 1-hydroxybenzotriazole (200 mg, 1.5 mmol, 1.1 eq.) was added. The reaction mixture was cooled to 0°C and 1,3-dicyclohexylcarbodiimide (310 mg, 1.5 mmol, 1.1 eq.) was added over 10 minutes. The reaction mixture was warmed to room temperature and stirred for 36 hours. It was then acidified with a 5% citric acid aqueous solution. The layers were separated and the organic layer washed with a 5% sodium bicarbonate aqueous solution (5 ml) and water (2 ml). The organic layer was dried (Na₂SO₄) and concentrated under reduced pressure. Purification by flash chromatography (SiO₂) eluting with cyclohexane/ethyl acetate/methanol 60:40:1 (v/v/v) afforded an intermediate compound (350 mg, 0.75 mmol, 55% yield). The latter (300 mg, 0.6 mmol, 1 eq.) was dissolved in THF (10 ml) under an inert nitrogen atmosphere.

Burgess reagent (160 mg, 0.7 mmol, 1.1 eq.) was added and the reaction mixture was heated to reflux temperature for 3 hours. Water (5 ml) was added. The product was extracted with ethyl acetate (2 x 15 ml), dried (Na₂SO₄) and concentrated under reduced pressure and purified by flash chromatography (SiO₂) eluting with cyclohexane/ethyl acetate/methanol 50:50:1 (v/v/v) to give 2-[4-(3,5-dichloro-pyridin-4-ylmethyl)-7-methoxy-phthalazin-1-yl]-4,5-dihydro-oxazole-4-carboxylic acid ethyl ester. This intermediate ester (100 mg, 0.22 mmol, 1 eq.) was dissolved in dichloromethane (3 ml) and the resulting solution was cooled to 0°C. 1,8-diazabicyclo[5.4.0]undec-7-ene (0.036 ml, 0.24 mmol, 1.1 eq.) and bromotrichloromethane (0.024 ml, 0.24 mmol, 1.1 eq.) were added dropwise. The reaction mixture was stirred at 0°C for 6 hours. An ammonium chloride saturated aqueous solution (10 ml) was added and the layers were separated. The combined organic layer was washed with brine (5 ml), dried (Na₂SO₄) and concentrated under reduced pressure. The resulting ester (85 mg, 0.18 mmol, 1 eq.) was then dissolved in a 3:1 (v/v) mixture of THF/water (6 ml) and lithium hydroxide (14 mg, 0.5 mmol, 3 eq.) was added. After 2 hours, a 5% citric acid aqueous solution (5 ml) was added. The product was extracted with ethyl acetate (2 x 15 ml), dried (Na₂SO₄) and concentrated under reduced pressure and the subsequent recrystallization from methanol afforded compound 9 as a white solid (65 mg, 0.14 mmol, 80% yield).
MS: 431 [M+H]⁺
NMR DMSO_d6: 9.08 (s, 1H, O-*CH=C); 8.86 (d, 1H, JHH=2.5 Hz, C=*CH-C); 8.71 (s, 2H, Py); 8.63 (d, 1H, JHH=9.3 Hz, *CH=CH-C-OMe); 7.87 (dd, 1H, CH=*CH-C-OMe); 5.08 (s, 2H, *CH2-Py); 4.04 (s, 3H, OMe).

### Example 15

### Synthesis of 2-[4-(3,5-dichloro-pyridin-4-ylmethyl)-7-methoxy-phthalazin-1-yl]-thiophene-3-carboxylic acid (compound 10)

Compound 10 was synthetised, operating as described for the compound 1, using as substrates intermediate 1 (500 mg, 1.4 mmol) and 3-formyl-2-thiopheneboronic acid (220 g, 1.4 mmol). The coupling reaction product was extracted with ethyl acetate (2 x 15 ml), dried (Na₂SO₄) and concentrated under reduced pressure and purified by flash chromatography (SiO₂) eluting with cyclohexane/ethyl acetate/methanol 60:40:1 (v/v/v) to give the intermediate compound 2-[4-(3,5-dichloro-pyridin-4-ylmethyl)-7-methoxy-phthalazin-1-yl]-thiophene-3-carboxyaldehyde (150 mg, 0.35 mmol, 25% yield). This intermediate aldehyde (80 mg, 0.18 mmol) was then oxidized to the corresponding acid operating as described for compound 5. Purification by flash chromatography (SiO₂) eluting with dichloromethane/methanol/acetic acid 95:5:95 (v/v/v) and subsequent recrystallization from dichloromethane/methanol 99:1 (v/v) afforded compound 10 as a white solid (40 mg, 0.09 mmol, 50% yield).
MS: 446 [M+H]⁺
NMR DMSO_d6: 8.70 (s, 2H, Py); 8.58 (d, 1H, JHH=9.1 Hz, *CH=CH-C-OMe); 7.97 (d ,1H, JHH=4.1 Hz, CH-S); 7.85-7.80 (m, 2H, S-CH=*CH, CH=*CH-C-OMe); 7.75 (d, 1H, JHH=2.5 Hz, C-*CH=C); 5.02 (s, 2H, *CH2-Py); 4.03 (s, 3H, OMe).

### Example 16

### Synthesis of N-methyl-D-glucamine salt of compound 2 (compound 11)

To a suspension of compound 2 (500 mg, 1.136 mmol) in methanol (5 ml) heated to reflux temperature under an inert nitrogen atmosphere, a solution of N-methyl-D-glucamine (233 mg, 1.19 mmol) in methanol (1.2 ml) and water (1.5 ml) was added. After complete dissolution the mixture was concentrated under reduced pressure at room temperature. Ethanol (10 ml) was added and the solution was maintained at 4°C for 4 days. The precipitate was filtered and washed with ethanol (5 ml) and diethyl ether (5 ml). It was dried under vacuum for 15 hours at 50°C to give compound 11 as pale yellow solid (400 mg, 0.61 mmol, 54% yield)
NMR DMSO_d6: 8.68 (s, 2H, Py); 8.54 (d, 1H, JHH=8.7 Hz, *CH=CH-C-OMe); 8.17 (s-broad, 1H, C=*CH-C-CO2H); 8.06 (d-broad, 1H, JHH=8.1 Hz, *CH), 7.78 (d-broad, 1H, JHH=8.1 Hz, *CH); 7.72 (dd, 1H, CH=*CH-C-OMe); 7.58-7.50 (m, 1H, CH=*CH-CH); 7.29 (d, 1H, JHH=2.3 Hz, C=*CH-C); 5.04 (s, 2H, *CH2-Py); 3.91-3.82 (m, 4H, OMe, *CHH-OH); 3.58-3.33 (m, 5H, HO-*CHH-*CH(OH)-*CH(OH)-*CH(OH)-*CH(OH)), 3.06-2.84 (m, 2H, CH₂-NH), 2.09 (s, 3H, NHMe).

### Example 17

### Synthesis of 2-amino-2-hydroxymethyl-propane-1,3-diol salt of compound 2 (compound 12)

To a suspension of compound 2 (500 mg, 1.136 mmol) in ethanol (5 ml) heated to reflux temperature under an inert nitrogen atmosphere, 2-amino-2-hydroxymethyl-propane-1,3-diol (138 mg, 1.13 mmol) was added. After complete dissolution the temperature was decreased to room temperature. After 2 hours the precipitate was filtered and washed with ethanol (5 ml) and diethyl ether (5 ml). It was dried under vacuum for 15 hours at 50°C to give compound 12 as pale yellow solid (400 mg, 0.71 mmol, 63% yield)
NMR DMSO_d6: 8.68 (s, 2H, Py); 8.54 (d, 1H, JHH=8.7 Hz, *CH=CH-C-OMe); 8.17 (s-broad, 1H, C=*CH-C-CO₂H); 8.05 (d-broad, 1H, JHH=8.1 Hz, *CH), 7.79 (d-broad, 1H, JHH=8.1 Hz, *CH); 7.77-7.70 (m, 1H, CH=*CH-C-OMe); 7.60-7.51 (m, 1H, CH=*CH-CH); 7.29 (d, 1H, JHH=2.3 Hz, C=*CH-C); 5.01 (s, 2H, *CH2-Py); 3.86 (s, 3H, OMe), 3.46 (s, 6H, C(CH₂)3).

### Example 18

### PDE 4 enzyme inhibition

### a) PDE 4 enzyme purification

PDE 4 enzyme was isolated from U937 cell line according to the method of Nielson et al. (J. Allergy Clin. Immunol. 1990, vol. 86, pages 801-807) partially modified for the FPLC technique (Fast Protein Liquid Chromatography).

U-937cell line (Istituto Zooprofilattico Sperimentale, Brescia, Italy) was maintained in RPMI 1640, with 10% foetal calf serum and 2 mM glutamine, at a cell density between 1×10⁶ and 8×10⁶ cells per ml in an incubator at 37°C with 5% CO₂.

U937 suspension was homogenised in a buffer containing 10 mM TRIS (tri-(hydroxymethyl)-aminomethane), 5 mM MgCl2, 4 mM EGTA (ethyleneglycol-bis-(β-aminoethylether)-N,N,N'N'-tetra-acetic acid), 5 mM □-mercaptoethanol, 1 µM leupeptin, 1 µM pepstatin, 1% TRITON x-100, and 100 µM phenylmethyl sulfonyl fluoride (PMSF) at pH 7.8.

The homogenate was centrifuged and the supernatant was used for the purification of the PDE 4 enzyme; it was seeded on a column connected to a biologic chromatography system (FPLC, BIO RAD).

PDE 4 enzyme was eluted with a linear gradient, from 0.05 M to 1 M of sodium acetate, and elution fractions were collected for PDE4 activity assay.

The fractions containing PDE4 activity were collected and, after an overnight dialysis against water to remove sodium acetate, were concentrated to 30% volume with an Amicon filtration system (using YM10 membrane filter).

Ethylene glycol (30% v/v) was added and the sample was stored at -20°C in a single aliquot until the use.

### b) PDE 4 activity assay

PDE 4 activity assay was performed using Scintillation Proximity Assay (SPA) Amersham kit consisting of tracer ³H-cAMP, PDE assay buffer (10 x solution: 500 mM TRIS/HCl pH 7.5, 83 mM MgCl₂ and 17 mM EGTA) and Yttrium SPA PDE beads (containing 18 mM zinc sulphate).

The radioactivity of the beads was measured using a scintillation counter (Packard model MINAXI β TRI-CARB 4000 SERIES).

The IC₅₀ values were calculated from concentration-inhibition curves by non linear regression analysis using the program ORIGIN 3.5 (MICROCAL SOFTWARE INC.) and each fitting was the mean ± SEM of 3 experiments using different PDE 4 preparations.

The compounds of formula I of the present invention showed to selectively inhibit PDE 4 enzyme.

The results are reported in the following table 1.

**Table 1**

| **Compound** | **IC 50 (nM)** |
|---|---|
| 1 | 25% at 10⁻⁷ |
| 2 | 37 |
| 3 | 47% at 10⁻⁷ |
| 4 | 16% at 10⁻⁷ |
| 5 | 91.3 |
| 6 | 45 |
| 7 | 54% at 10⁻⁷ |
| 8 | 23 |
| 9 | 14% at 10⁻⁷ |
| 10 | 48 |

### Example 19

### TNFα release in whole human blood

The blood samples obtained from healthy volunteers were collected in heparinised tubes and diluted 1:5 with RPMI 1640 without serum addition.

The test was carried out in 96-well plates and samples containing 150 µl of diluted blood and 150 µl of RPMI 1640 with control vehicle or with different concentration of the compounds of the present invention were incubated at 37°C in a 5% CO₂ humidified atmosphere for 30 minutes.

The whole blood in the assay samples was diluted 1:10 (v/v).

The samples were stimulated with LPS (lipopolysaccharide from E. Coli: serotype B 0:55, Sigma) 0.25 µg/ml and incubated for 24 hours.

After centrifugation, the supernatants were collected for TNFα levels determination by a commercially available ELISA kit (Biosource).

The test objects were dissolved in DMSO at 10⁻² M and further diluted in RPMI 1640.

The final concentration of DMSO did not exceed 0.1% and did not affect TNFα release.

Each compound was tested in duplicate at nine concentrations and the data obtained were further confirmed using blood samples from different donors.

The percent inhibition of TNFα production at each concentration was calculated and IC₅₀ was determined using a 4-parameters logistic equation (Origin calculation program, Microcal Software Inc.)

The compounds of formula I of the present invention showed to inhibit the TNFα release.

The results, expressed as IC 50 at two different concentration, are reported in the following table 2.

**Table 2**

| **Compound** | **IC 50 (nM)** | **IC 50 (nM)** |
|---|---|---|
| | **h-WB 1:10** | **h-WB** |
| 2 | 2470 | 33000 |
| 8 | 1230 | 14300 |
| 10 | 18000 | |

### Example 20

### PDE 3 and PDE 5 enzyme inhibition

### a) PDE 3 and PDE 5 enzyme purification

PDE 3 and PDE 5 enzymes were purified from platelet rich plasma (PRP) obtained from healthy volunteers.

PDE 3 and PDE 5 enzymes were purified according to Simpson A.W.M. et al. (Biochem. Pharmacol. 1988, 37, 2315-2320) partially modified for the FPLC technique (Fast Protein Liquid Chromatography).

The PRP was diluted 1:2 in saline solution and centrifuged at 2000 x g for 15 minutes at room temperature.

The pellet was suspended in 10 ml of lysis solution (155 mM NH₄Cl, 10 mM KHCO₃ and 0.1 mM Na₂EDTA pH 7.4) and incubated for 10 minutes on ice-bath to remove erythrocyte contamination.

After centrifugation, platelets were suspended in 10 ml of 20 mM Bis-Tris, 2 mM EDTA, 5 mM β-mercaptoethanol, 50 mM sodium acetate, 2 mM benzamidine) and homogenised with a Polytron homogeniser on ice-bath.

The homogenate was centrifuged and the supernatant applied to a UNO Q12 column connected to a Biologic Chromatography system (FPLC, BIO RAD).

PDE 3 and PDE 5 enzymes were eluted with a linear 0.05-1M sodium acetate gradient and the elution fractions were collected for PDE enzymes activity assay.

Fractions containing PDE activities were pooled, dialysed overnight against distilled water and concentrated 10 times by an Amicon filtration system (using YM10 membrane filter).

Ethylene glycol was added to a final concentration of 30% v/v and the solution stored at -20°C.

Enzymatic activity was stable for several weeks under these conditions.

### b) PDE 3 and PDE 5 activity assay

Enzymes activity assay were performed using Scintillation Proximity Assay (SPA) Amersham kit consisting of tracer ³H-cAMP for PDE3 or ³H-cGMP for PDE5, PDE assay buffer (10x solution: 500 mM TRIS/HCl pH 7.5, 83 mM MgCl₂ and 17 mM EGTA) and Yttrium SPA PDE beads (containing 18 mM zinc sulphate).

The radioactivity of the beads was measured using a scintillation counter (Packard model MINAXI β TRI-CARB 4000 SERIES).

The results, expressed as IC 50 of some compounds which are representative of the entire class of compounds, are reported in the following table 3.

**Table 3**

| **Compound** | **PDE 3-5** |
|---|---|
| | **IC 50 (nM)** |
| 2 | >10000 |
| 8 | >10000 |
| 10 | >10000 |

### Example 21

### PDE 2 enzyme inhibition

### a) PDE 2 enzyme purification

PDE 2 was purified from murine PC12 cells according to Whalin et al. (Molecular Pharmacol. 1991, 39, 711-717).

PC12 cells from Istituto Zooprofilattico Brescia (Italy) were maintained in 150 cm³ flasks at 37 °C and 5% CO₂ in DMEM containing 5% FCS, 15% horse serum, 1% penicillin, 1% streptomycin and 1% glutamine.

The cells were expanded paying attention to split them only at reached confluency.

After washing the cells were detached with 0.05% trypsin/0.02%EDTA and collected in a clean test tube.

After centrifugation, the cells were suspended in buffer containing Bis-Tris 20 mM, EDTA 2 mM, 2-mercaptoethanol 5 mM, leupeptin 1 µM, pepstatin A 1 µM, PMSF 100 µM, pH 6.5, and homogenised using a Polytron homogeniser.

After centrifugation, the supernatant fraction was applied to a UNO Q-12 column connected to a Biologic Chromatography system (FPLC, BIO RAD).

PDE 2 enzyme was eluted with a linear 0.05-1M sodium acetate gradient and elution fractions were collected for PDE 2 enzyme activity assay.

Fractions containing PDE 2 enzyme activity were pooled, dialysed overnight against distilled water and concentrated 10 times by an Amicon filtration system (using YM10 membrane filter).

Ethylene glycol was added to a final concentration of 30% v/v and the solution stored at -20°C.

Enzymatic activity was stable for several weeks under these conditions.

### b) PDE 2 activity assay

PDE 2 activity assay was performed using Scintillation Proximity Assay (SPA) Amersham kit consisting of tracer ³H-cAMP, PDE assay buffer (10 x solution: 500 mM TRIS/HCl pH 7.5, 83 mM MgCl₂ and 17 mM EGTA) and Yttrium SPA PDE beads (containing 18 mM zinc sulphate).

The radioactivity of the beads was measured using a scintillation counter (Packard model MINAXI β TRI-CARB 4000 SERIES).

The results, expressed as IC 50 of some compounds, which are representative of the entire class of compounds, are reported in the following table 4.

**Table 4**

| **Compound** | **PDE 2** |
|---|---|
| | **IC 50 (nM)** |
| 2 | >10000 |
| 8 | >10000 |
| 10 | >10000 |

### Example 22

### PDE 1 enzyme inhibition

### a) PDE 1 enzyme purification

Lyophilised PDE 1 enzyme, partially purified from bovine heart, was purchased from Sigma (P0520).

The enzyme consists of the P1 fraction reported by Ho et al. (Biochim Biophys Acta, 1976, vol. 429(2), 461-473).

The lyophilised enzyme was reconstituted with 30% v/v ethylene glycol at a concentration of 500g/ml and stored at -20°C.

### b) PDE 1 activity assay

PDE 1 activity was determined using the Scintillation Proximity Assay (SPA) Amersham kit consisting of tracer ³H-cAMP, PDE assay buffer (10 x solution: 500 mM TRIS/HCl pH 7.5, 83 mM MgCl₂ and 17 mM EGTA) and Yttrium SPA PDE beads (containing 18 mM zinc sulphate).

The radioactivity of the beads was measured using a scintillation counter (Packard model MINAXI β TRI-CARB 4000 SERIES).

The results, expressed as IC 50 of some compounds, which are representative of the entire class of compounds, are reported in the following table 5.

**Table 5**

| **Compound** | **PDE 1** |
|---|---|
| | **IC 50 (nM)** |
| 2 | >10000 |
| 8 | >10000 |
| 10 | >10000 |

### Example 23

The data concerning the solubility of the compounds of formula I of the present invention in comparison with those exemplified in the international patent application WO 00/05218 are hereinafter reported.

HPLC method used to test the solubility.

Solubility test was performed by HPLC analysis (column 4.6 X 15mm) water/0.1% TFA - CH₃CN/0.06% TFA gradient) against standard.

A 10 M solution of the substance dissolved in DMSO was prepared.

In order to prepare the sample 190 µl of PBS (Phosphate Buffer Saline, pH 7.4) were added to 10 µl of the above solution; instead (while) in order to prepare the standard 190 µl of DMSO to other 10 µl of solution were added.

The results obtained for the compounds of formula I are reported in the following table 6.

**Table 6**

| **Compound** | **MW** | **Sol. (mg/ml)** | **Sol. (µM)** |
|---|---|---|---|
| 2 | 448 | 0.216 | 482.14 |
| 1 | 440 | 0.218 | 495.45 |
| 6 | 533 | 0.271 | 508.44 |
| 3 | 485 | 0.298 | 614.43 |
| 4 | 470 | 0.226 | 480.85 |
| 5 | 470 | 0.23 | 489.36 |
| 7 | 470 | 0.235 | 500.00 |
| 8 | 476 | 0.47 | 987.39 |
| 9 | 431 | 0.202 | 468.68 |
| 10 | 446 | 0.253 | 567.26 |

The results obtained for the compounds of the international patent application WO 00/05218 in the following table 7 are reported. 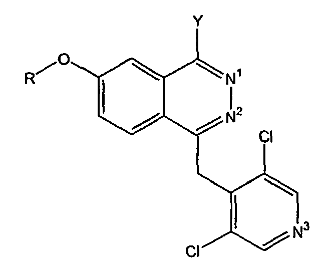 R is a -CH₃ group when not differently specified.

**Table 7**

| **Y** | **Sol. (µM)** | **compound** |
|---|---|---|
| H | 30 | 1 |
| H, N¹-ox | 40 | 2 |
| H, N¹-ox, N³-ox | 52 | 3 |
| Ethyl | 37 | 4 |
| phenyl | 24 | 5 |
| 4-phenyl-butyl | <1 | 6 |
| thiazol-2-yl | <1 | 26 |
| phenylethynyl | 6 | 27 |
| Pyrrolidin-1-one | 130 | 28 |
| Triazol-1-yl | 12 | 29 |
| Morpholin-4-yl | 87 | 38 |
| Propylamino | 52 | 39 |
| Dimethylamino | 58 | 44 |
| Imidazol-1-yl | 20 | 45 |
| Thiazol-2-yl-amino | 3 | 46 |
| phenoxy | 6 | 47 |
| 4-methyl-piperazin-1-yl | 71 | 48 |
| Pyrrolidin-1-yl | 63 | 49 |
| Triazol-1-yl, N¹-ox | 37 | 52 |
| Triazol-1-yl, R=-CHF₂ | 10 | 54 |
| Morpholin-4-yl, R=-CHF₂ | 82 | 55 |
| Morpholin-4-yl-methyl | 39 | 56 |
| Pyrrolidin-1-yl-methyl | 37 | 57 |
| 3-morpholin-4-yl-propynyl | 13 | 58 |
| 2-methyl-butyn-2-ol | 33 | 59 |
| Ethynyl | 32 | 60 |
| -CONH₂ | 7 | 61 |
| Carbonitrile | 5 | 62 |
| Pyrrolidine carbox. | >200 | 75 |
| Thiazol-2-yl, N³-ox | <1 | 85 |

The compound identified in the international patent application WO 00/05218 with number 75 is endowed with a PDE 4 inhibitory activity lower than that reported for the compounds of the present invention.

### Example 24

### Pharmacokinetic study in rats

A mixture containing 3-5 compounds according to what described by Ward K.W. et al. (Drug Metab. Dispos. 2001, vol. 29, 82-88) was administered to rats at 10 mg/kg dosage by oral route (1 mg/ml in 77% N-methylglucosamine 25 mM and 20% PG containing 3%

Tween 80) or 3 mg/kg intravenously (3 mg/ml in 15% DMSO/85% PEG).

The animals received only one treatment per study.

The rats permanently implanted with jugular catheter (Silastic^{R}, Dow Corning) were treated with the test mixture by an intravenous bolus (via implanted catheter) or by oral gavage.

Blood samples were collected from the jugular catheter at different times up to 6 h after administration.

Plasma samples were analyzed after protein precipitation by two volumes of CH₃CN and plasma concentrations were determined by liquid chromatography/mass spectrometry.

The results obtained with some of the compounds of formula I are reported in table 8.

**Table 8**

| **Compound** | **PK rat** | **PK rat** |
|---|---|---|
| | **t/2 (min.)** | **%F** |
| 2 | 132 | 43% |
| 8 | 66 | 41 % |
| 10 | 21 | 7% |

The increase of the solubility of the compounds of the invention allowed to perform the bioavailability studies by means of physiologic vehicles normally used in the most common pharmaceutical compositions.

The performed tests by means of a physiologic vehicle on the compounds comprised in the general formula of international patent application WO 00/05218 showed bioavailability values equal to zero.

Being poor the solubility of the compounds comprised in the general formula of the international patent application WO 00/05218, it was necessary to performe the pharmacokinetic studies using a 15% DMSO / 85% PEG solution of the compounds both for intravenous and for oral route, i.e. by an absolutely non physiological vehicle.

The following table 9 reports the bioavailability values obtained in such conditions.

**Table 9**

| **Compound** | **PK rat** |
|---|---|
| | **%F** |
| 5 | 29% |
| 20 | 11% |
| 15 | 0% |
| 17 | 8% |
| 29 | 27% |
| 60 | 0% |
| 49 | 9% |

Therefore, the compounds of formula I object of the present invention when administered in a physiological vehicle showed good bioavailability values comparable and in many cases higher than the compounds comprised in the general formula of the patent application of which they are a selection, underlying that the bioavailability of these last ones was measured in non physiological DMSO / PEG solution.

## Claims

1. A compound of formula 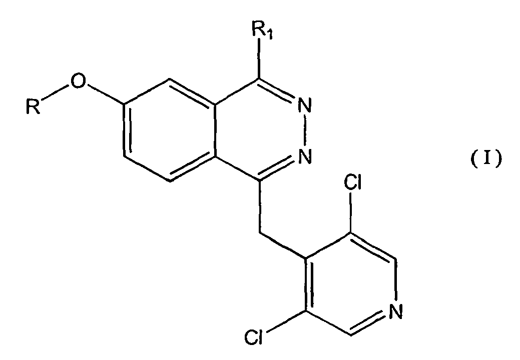 wherein
R is methyl or a difluoromethyl group;
R₁ is phenyl or a 5 or 6 membered aromatic heterocycle containing from 1 to 3 heteroatoms selected among nitrogen, oxygen and sulphur and bonded to the phthalazine nucleus by a carbon-carbon bond, both the phenyl and the heterocycle being substituted with a carboxy group and optionally with a second functional group selected among methoxy, nitro, N-acetylamino, N-methanesulfonyl-amino;
the N-oxidised derivatives of the compounds of formula I and the pharmaceutically acceptable salts thereof.

2. A compound according to claim 1 wherein R₁ is phenyl substituted with a carboxy group and optionally with a second functional group selected among methoxy, nitro, N-acetylamino, N-methanesulfonyl-amino.

3. A compound acording to claim 2 wherein R₁ is phenyl substituted with a carboxy group and particularly the carboxy group is in meta position with regard to the phthalazine nucleus.

4. A compound according to claim 1 wherein R₁ is a 5 or 6 membered aromatic heterocycle containing from 1 to 3 heteroatoms selected among nitrogen, oxygen and sulphur and bonded to the phthalazine nucleus by a carbon-carbon bond, substituted with a carboxy group and optionally with a second functional group selected among methoxy, nitro, N-acetylamino, N-methanesulfonyl-amino;

5. A compound according to claim 1 selected from:
3-[4-(3,5-Dichloro-pyridin-4-ylmethyl)-7-methoxy-phthalazin-1-yl]-benzoic acid;
4-[4-(3,5-Dichloro-pyridin-4-ylmethyl)-7-methoxy-phthalazin-1-yl]-benzoic acid;
2-[4-(3,5-Dichloro-pyridin-4-ylmethyl)-7-methoxy-phthalazin-1-yl]-benzoic acid;
3-[4-(3,5-Dichloro-pyridin-4-ylmethyl)-7-methoxy-phthalazin-1-yl]-5-nitro-benzoic acid;
5-[4-(3,5-Dichloro-pyridin-4-ylmethyl)-7-methoxy-phthalazin-1-yl]-2-methoxy-benzoic acid;
3-[4-(3,5-Dichloro-pyridin-4-ylmethyl)-7-methoxy-phthalazin-1-yl]-4-methoxy-benzoic acid;
3-[4-(3,5-Dichloro-pyridin-4-ylmethyl)-7-methoxy-phthalazin-1-yl]-5-methanesulfonyl-amino-benzoic acid;
3-Acetylamino-5-[4-(3,5-dichloro-pyridin-4-ylmethyl)-7-methoxy-phthalazin-1-yl]-benzoic acid;
3-[4-(3,5-Dichloro-pyridin-4-ylmethyl)-7-methoxy-phthalazin-1-yl]-5-methoxy-benzoic acid;
3-[4-(3,5-Dichloro-pyridin-4-ylmethyl)-7-difluoromethoxy-phthalazin-1-yl]-benzoic acid;
2-[4-(3,5-Dichloro-pyridin-4-ylmethyl)-7-methoxy-phthalazin-1-yl]-oxazole-4-carboxylic acid;
2-[4-(3,5-Dichloro-pyridin-4-ylmethyl)-7-methoxy-phthalazin-1-yl]-thiophene-3-carboxylic acid;
(2-amino-2-hydroxymethyl-propane-1,3-diol) salt of 3-[4-(3,5-Dichloro-pyridin-4-ylmethyl)-7-methoxy-phthalazin-1-yl]-benzoic acid;
N-methyl-glucamine salt of 3-[4-(3,5-Dichloro-pyridin-4-ylmethyl)-7-methoxy-phthalazin-1-yl]-benzoic acid;

6. A process for the preparation of a compound according to claim 1 by an aromatic nucleophilic substitution reaction or a coupling reaction, in presence of a catalyst such as for example palladium, between a compound of formula 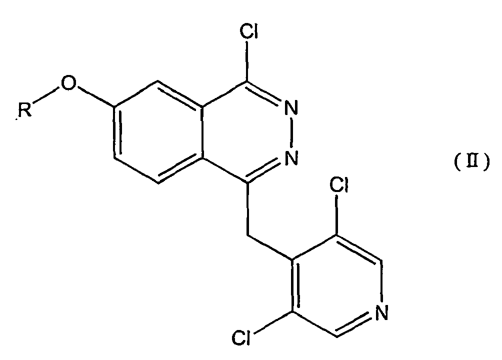 wherein
R has the meanings reported for the compounds of formula I;
and a reactant such as a tin or boronic acid derivative, suitable for the substitution of the halogen atom directly bonded to the phthalazine nucleus with a phenyl or a heterocycle substituted with a carboxy group and optionally with a second functional group defined in the meanings of R₁ in formula I.

7. A process according to claim 6 wherein the coupling reaction is carried out between the compounds of formula II and the proper boronic acid in presence of palladium, triphenylphosphine and an aqueous solution of potassium carbonate.

8. A pharmaceutical composition containing a therapeutically effective amount of a compound according to claim 1 in admixture with a pharmaceutically acceptable carrier.

9. A pharmaceutical composition according to claim 8 for the treatment of allergic and inflammatory diseases

10. A pharmaceutical composition according to claim 8 for the treatment of respiratory diseases.

## Patentansprüche

1. Verbindung der Formel 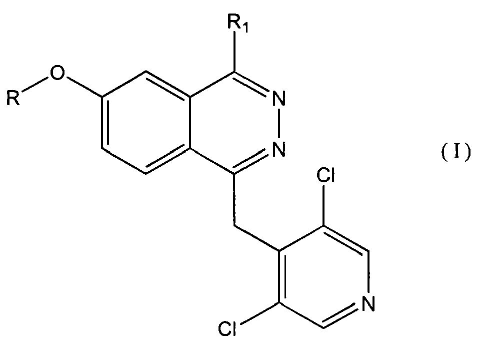 wobei
R Methyl oder eine Difluormethylgruppe ist;
R₁ Phenyl oder ein 5- oder 6-gliedriger aromatischer Heterocyclus ist, der 1 bis 3 Heteroatome enthält, die aus Stickstoff, Sauerstoff und Schwefel ausgewählt, und an den Phthalazinnucleus durch eine Kohlenstoff-Kohlenstoff-Bindung gebunden sind, wobei sowohl Phenyl als auch Heterocyclus mit einer Carboxylgruppe und optional mit einer zweiten funktionellen Gruppe substituiert sind, die aus einer Methoxy-, Nitro-, N-Acetylamino-, N-Methansulfonylaminogruppe ausgewählt ist;
die N-oxidierten Derivate der Verbindung aus Formel I und die pharmazeutisch akzeptablen Salze davon.

2. Verbindung nach Anspruch 1, wobei R₁ Phenyl ist, das mit einer Carboxylgruppe und optional mit einer zweiten funktionellen Gruppe substituiert ist, die aus einer Methoxy-, Nitro-, N-Acetylamino-, N-Methansulfonylaminogruppe ausgewählt ist.

3. Verbindung nach Anspruch 2, wobei R₁ Phenyl ist, das mit einer Carboxylgruppe substituiert ist, und wobei sich die Carboxylgruppe insbesondere in meta-Stellung, bezogen auf den Phthalazinnucleus, befindet.

4. Verbindung nach Anspruch 1, wobei R₁ ein 5- oder 6-gliedriger aromatischer Heterocyclus ist, der 1 bis 3 Heteroatome enthält, die aus Stickstoff, Sauerstoff und Schwefel ausgewählt, und an den Phthalazinnucleus durch eine Kohlenstoff-Kohlenstoff-Bindung gebunden sind, der mit einer Carboxylgruppe und optional mit einer zweiten funktionellen Gruppe substituiert ist, die aus einer Methoxy-, Nitro-, N-Acetylamino-, N-Methansulfonylaminogruppe ausgewählt ist;

5. Verbindung nach Anspruch 1, ausgewählt aus:
3-[4-(3,5-Dichlor-pyridin-4-ylmethyl)-7-methoxy-phthalazin-1-yl]-benzoesäure;
4-[4-(3,5-Dichlor-pyridin-4-ylmethyl)-7-methoxy-phthalazin-1-yl]-benzoesäure;
2-[4-(3,5-Dichlor-pyridin-4-ylmethyl)-7-methoxy-phthalazin-1-yl]-benzoesäure;
3-[4-(3,5-Dichlor-pyridin-4-ylmethyl)-7-methoxy-phthalazin-1-yl]-5-nitrobenzoesäure;
5-[4-(3,5-Dichlor-pyridin-4-ylmethyl)-7-methoxy-phthalazin-1-yl]-2-methoxybenzoesäure;
3-[4-(3,5-Dichlor-pyridin-4-ylmethyl)-7-methoxy-phthalazin-1-yl]-4-methoxybenzoesäure;
3-[4-(3,5-Dichlor-pyridin-4-ylmethyl)-7-methoxy-phthalazin-1-yl]-5-methansulfonyl-amino-benzoesäure;
3-Acetylamino-5-[4-(3,5-dichlor-pyridin-4-ylmethyl)-7-methoxy-phthalazin-1-yl]-benzoesäure;
3-[4-(3,5-Dichlor-pyridin-4-ylmethyl)-7-methoxy-phthalazin-1-yl]-5-methoxybenzoesäure;
3-[4-(3,5-Dichlor-pyridin-4-ylmethyl)-7-difluormethoxy-phthalazin-1-yl]-benzoesäure;
2-[4-(3,5-Dichlor-pyridin-4-ylmethyl)-7-methoxy-phthalazin-1-yl]-oxazol-4-carbonsäure;
2-[4-(3,5-Dichlor-pyridin-4-ylmethyl)-7-methoxy-phthalazin-1-yl]-thiophen-3-carbonsäure;
(2-Amino-2-hydroxymethyl-propan-1,3-diol)-Salz von 3-[4-(3,5-Dichlor-pyridin-4-ylmethyl)-7-methoxy-phthalazin-1-yl]-benzoesäure;
N-Methylglucaminsalz von 3-[4-(3,5-Dichlor-pyridin-4-ylmethyl)-7-methoxy-phthalazin-1-yl]-benzoesäure;

6. Verfahren zur Herstellung einer Verbindung nach Anspruch 1 durch eine aromatische nucleophile Substitutionsreaktion oder eine Kupplungsreaktion in Gegenwart eines Katalysators, wie zum Beispiel Palladium, zwischen einer Verbindung der Formel 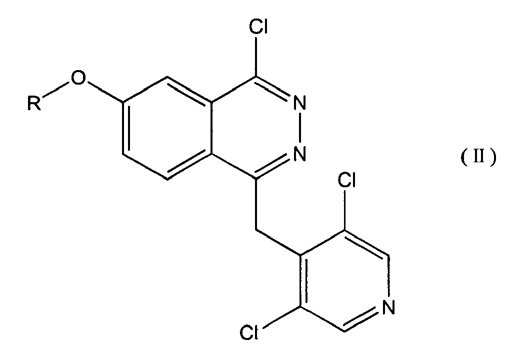 wobei
R die Bedeutungen hat, die für die Verbindungen der Formel I berichtet wurden;
und ein Reaktant, wie etwa Zinn oder ein Boronsäurederivat, der für die Substitution des Halogenatoms geeignet ist, das direkt an den Phthalazinnucleus gebunden ist, mit einem Phenyl oder einem Heterocyclus, der mit einer Carboxylgruppe und optional mit einer zweiten funktionellen Gruppe substituiert ist, die in den Bedeutungen von R₁ in Formel I definiert ist.

7. Verfahren nach Anspruch 6, wobei die Kopplungsreaktion zwischen den Verbindungen der Formel II und der passenden Boronsäure in Gegenwart von Palladium, Triphenylphosphin und einer wässrigen Kaliumcarbonatlösung durchgeführt wird.

8. Pharmazeutische Zusammensetzung, die eine therapeutisch wirksame Menge einer Verbindung nach Anspruch 1 in Beimischung mit einem pharmazeutisch akzeptablen Träger enthält.

9. Pharmazeutische Zusammensetzung nach Anspruch 8 zur Behandlung von allergischen und entzündlichen Erkrankungen.

10. Pharmazeutische Zusammensetzung nach Anspruch 8 zur Behandlung von Erkrankungen der Atemwege.

## Revendications

1. Composé de formule : 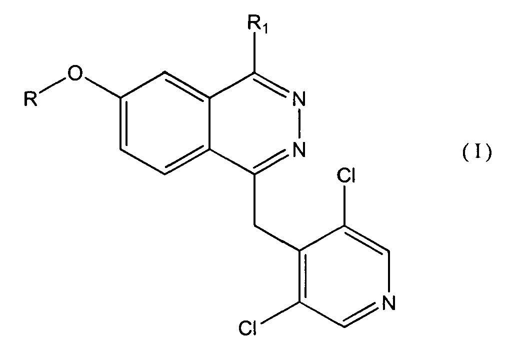 dans laquelle:
R est un groupe méthyle ou difluorométhyle;
R₁ est un groupe phényle ou un hétérocycle aromatique à 5 ou 6 chaînons contenant de 1 à 3 hétéroatomes choisis parmi les atomes d'azote, d'oxygène et de soufre et liés au noyau phtalazine par une liaison carbone-carbone, à la fois le groupe phényle et l'hétérocycle étant substitués par un groupe carboxy et facultativement par un second groupe fonctionnel choisi parmi les groupes méthoxy, nitro, N-acétylamino, N-méthanesulfonyl-amino;
les dérivés N-oxydés des composés de formule I et les sels pharmaceutiquement acceptables de ceux-ci.

2. Composé selon la revendication 1, dans lequel R₁ est un groupe phényle substitué par un groupe carboxy et facultativement par un second groupe fonctionnel choisi parmi les groupes méthoxy, nitro, N-acétylamino, N-méthanesulfonyl-amino.

3. Composé selon la revendication 2, dans lequel R₁ est un groupe phényle substitué par un groupe carboxy et particulièrement le groupe carboxy est en position méta par rapport au noyau phtalazine.

4. Composé selon la revendication 1, dans lequel R₁ est un hétérocycle aromatique à 5 ou 6 chaînons contenant de 1 à 3 hétéroatomes choisis parmi les atomes d'azote, d'oxygène et de soufre et liés au noyau phtalazine par une liaison carbone-carbone, substitué par un groupe carboxy et facultativement par un second groupe fonctionnel choisi parmi les groupes méthoxy, nitro, N-acétylamino, N-méthanesulfonyl-amino.

5. Composé selon la revendication 1, choisi parmi:
l'acide 3-[4-(3,5-dichloro-pyridin-4-ylméthyl)-7-méthoxy-phtalazin-1-yl]-benzoïque; l'acide 4-[4-(3,5-dichloro-pyridin-4-ylméthyl)-7-méthoxy-phtalazin-1-yl]-benzoïque
l'acide 2-[4-(3,5-dichloro-pyridin-4-ylméthyl)-7-méthoxy-phtalazin-1-yl]-benzoïque;
l'acide 3-[4-(3,5-dichloro-pyridin-4-ylméthyl)-7-méthoxy-phtalazin-1-yl]-5-nitro-benzoïque;
l'acide 5-[4-(3,5-dichloro-pyridin-4-ylméthyl)-7-méthoxy-phtalazin-1-yl]-2-méthoxy-benzoïque;
l'acide 3-[4-(3,5-dichloro-pyridin-4-ylméthyl)-7-méthoxy-phtalazin-1-yl]-4-méthoxy-benzoïque;
l'acide 3-[4-(3,5-dichloro-pyridin-4-ylméthyl)-7-méthoxy-phtalazin-1-yl]-5-méthanesulfonyl-amino-benzoïque;
l'acide 3-acétylamino-5-[4-(3,5-dichloro-pyridin-4-ylméthyl)-7-méthoxy-phtalazin-1-yl]-benzoïque;
l'acide 3-[4-(3,5-dichloro-pyridin-4-ylméthyl)-7-méthoxy-phtalazin-1-yl]-5-méthoxy-benzoïque;
l'acide 3-[4-(3,5-dichloro-pyridin-4-ylméthyl)-7-difluorométhoxy-phtalazin-1-yl]-benzoïque;
l'acide 2-[4-(3,5-dichloro-pyridin-4-ylméthyl)-7-méthoxy-phtalazin-1-yl]-oxazole-4-carboxylique;
l'acide 2-[4-(3,5-dichloro-pyridin-4-ylméthyl)-7-méthoxy-phtalazin-1-yl]-thiophène-3-carboxylique;
le sel (2-amino-2-hydroxyméthyl-propane-1,3-diol) de l'acide 3-[4-(3,5-dichloro-pyridin-4-ylméthyl)-7-méthoxy-phtalazin-1-yl]-benzoïque;
le sel N-méthyl-glucamine de l'acide 3-[4-(3,5-dichloro-pyridin-4-ylméthyl)-7-méthoxy-phtalazin-1-yl]-benzoïque.

6. Procédé de préparation d'un composé selon la revendication 1, par une réaction de substitution nucléophile aromatique ou une réaction de couplage, en présence d'un catalyseur tel que par exemple le palladium, entre un composé de formule: 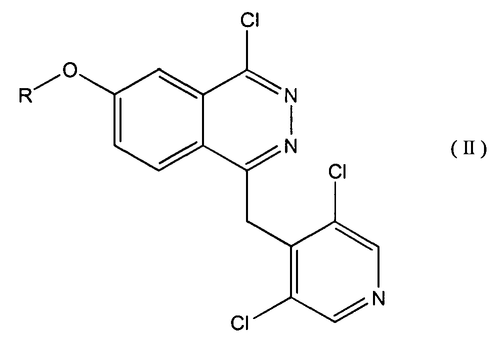 dans laquelle:
R a la signification rapportée pour les composés de formule I;
et un réactif tel qu'un dérivé de l'étain ou de l'acide boronique, adapté pour la substitution de l'atome d'halogène directement lié au noyau phtalazine par un groupe phényle ou un hétérocycle substitué par un groupe carboxy et facultativement par un second groupe fonctionnel défini dans la signification de R₁ dans la formule I.

7. Procédé selon la revendication 6, dans lequel la réaction de couplage est réalisée entre les composés de formule II et l'acide boronique lui-même en présence de palladium, de triphénylphosphine et d'une solution aqueuse de carbonate de potassium.

8. Composition pharmaceutique contenant une quantité thérapeutiquement efficace d'un composé selon la revendication 1 mélangé avec un vecteur pharmaceutiquement acceptable.

9. Composition pharmaceutique selon la revendication 8, destinée au traitement de maladies allergiques et inflammatoires.

10. Composition pharmaceutique selon la revendication 8, destinée au traitement de maladies respiratoires.
